# EUROPÄISCHE PATENTSCHRIFT

(11) **EP 2 945 925 B1**
(45) Veröffentlichungstag und Bekanntmachung des Hinweises auf die Patenterteilung: **01.11.2017**
(21) Anmeldenummer: 14701686.9
(22) Anmeldetag: 14.01.2014
(51) Int. Cl.: C07C 51/377, C07C 57/04

(54) **VERFAHREN ZUR HERSTELLUNG VON ACRYLSÄURE**
METHOD FOR PRODUCING ACRYLIC ACID
PROCÉDÉ DE PRÉPARATION DE L'ACIDE ACRYLIQUE

(30) Priorität: 16.01.2013 DE 102013000602
(43) Veröffentlichungstag der Anmeldung: 25.11.2015
(73) Patentinhaber: Evonik Degussa GmbH, 45128 Essen (DE)
(72) Erfinder: KUPPINGER, Franz-Felix, 45768 Marl (DE); KLASOVSKY, Florian, 45721 Haltern am See (DE); MAY, Alexander, 64342 Seeheim-Jugenheim (DE); OH, Min-Zae, 50968 Köln (DE)
(86) Internationale Anmeldenummer: PCT/EP2014/050550
(87) Internationale Veröffentlichungsnummer: WO 2014/111363

(56) Entgegenhaltungen:
- WO-A2-03/082795

## Beschreibung

Die vorliegende Erfindung betrifft ein Verfahren zur Herstellung von Acrylsäure durch Dehydratisierung einer Hydroxycarbonsäure, die 3 Kohlenstoffatome aufweist, durch in Kontakt bringen der Hydroxycarbonsäure bei einer Temperatur von größer 150 °C mit einer bei dieser Temperatur flüssigen Mischung, enthaltend mindestens ein Metallsalz einer Hydroxycarbonsäure, die 3 Kohlenstoffatome aufweist, und Wasser.

Acrylsäure ist ein bedeutendes Zwischenprodukt, das insbesondere in Form seiner Polymerisate Verwendung findet. Die bekannteste Verwendung von Acrylsäure ist die Herstellung von superabsorbierenden Polymeren auf Basis von Polyacrylsäure.

Die Herstellung von Acrylsäure erfolgt klassisch chemisch über die Oxidation von Propen zum Acrolein und anschließende weitere Oxidation zur Acrylsäure.

Neuere Verfahren versuchen Acrylsäure durch Verwendung anderer Rohstoffe herzustellen. So wird z. B. in WO 2008/061819 ein Verfahren zur Herstellung von (Meth)acrylsäure (die Bezeichnung (Meth)acrylsäure soll sowohl Methacrylsäure als auch Acrylsäure umfassen) beschrieben, bei dem cyclische Ester in Gegenwart eines Katalysators zu (Meth)acrylsäure umgesetzt werden.

In jüngerer Zeit sind auf Grund ökonomischer und ökologischer Erwägungen zahlreiche Versuche unternommen worden, andere Rohstoffe zur Herstellung der Acrylsäure oder Methacrylsäure einzusetzen, insbesondere nachwachsende Rohstoffe.

So beschreibt WO 2008/145737 die Herstellung von Methacrylsäure durch Dehydratisierung von 3-Hydroxyisobuttersäure, die biotechnologisch aus nachwachsenden Rohstoffen, insbesondere aus Kohlehydraten und/oder aus Glycerin hergestellt wurde. Die Dehydratisierung wird in flüssiger Phase bei einem Druck in einem Bereich von 200 bis 500 mbar, bei einer Temperatur in einem Bereich von 200 bis 230°C und in Gegenwart von Alkalimetall-Ionen als Katalysator durchgeführt.

DE-OS 1 768 253 beschreibt ebenso ein solches Verfahren zur Herstellung von Methacrylsäure durch Dehydratisierung von α-Hydroxyisobuttersäure (HIBS), welches dadurch gekennzeichnet ist, dass man HIBS in flüssiger Phase bei einer Temperatur von wenigstens 160°C in Gegenwart eines Dehydratisierungskatalysators umsetzt, der aus einem Metallsalz von alpha-Hydroxyisobuttersäure besteht. Besonders geeignet sind in diesem Fall die Alkali- und Erdalkalisalze von HIBS, die in einer HIBS-Schmelze durch Umsetzung geeigneter Metallsalze *in situ* hergestellt werden. Laut Patent werden Methacrylsäure-Ausbeuten bis 95% ex HIBS beschrieben, wobei der Feed der kontinuierlichen Verfahrensweise aus HIBS und ca. 1,5 Gew.-% HIBS-Alkalisalz besteht.

Beide vorgenannten Dokumente beschreiben keine Verfahren zur Herstellung von Acrylsäure. WO 03/082795 A2 zeigt ein Verfahren zur Erzeugung a,ß-ungesättigter Carbonsäuren durch Dehydratisierung von ß-Hydroxycarbonsäuren, bei dem Calcium-3-hydroxypropionat als Katalysator eingesetzt werden kann.

WO 2013/155245 der Procter & Gamble beschreibt die Verwendung von Mischungen von Phosphaten als Katalysatoren bei der Dehydratisierung von Milchsäure zu Acrylsäure in der Gasphase.

Zu Dehydratisierungen in der Gasphase müssen die Reaktanden zunächst in die Gasphase geebracht werden, was z. B. bei Einwirken von thermischer Energie auf die Reaktanden häufig zu einem Abbau der Reaktanden führt, was sich z. B. durch Verkokungen bemerkbar macht.

Aufgabe der vorliegenden Erfindung war deshalb die Bereitstellung eines Verfahrens zur Herstellung von Acrylsäure aus Hydroxycarbonsäuren, welches die Nachteile der Verfahren gemäß dem Stand der Technik vermeidet.

Überraschenderweise wurde gefunden, dass Acrylsäure mit einem Verfahren wie in den Ansprüchen beschrieben aus Hydroxycarbonsäure hergestellt werden kann.

Gegenstand der vorliegenden Erfindung ist deshalb ein Verfahren zur Herstellung von Carbonsäuren wie in den Ansprüchen beansprucht und in der nachfolgenden Beschreibung weiter beschrieben.

Das erfindungsgemäße Verfahren hat den Vorteil, dass Acrylsäure unter Verwendung von Milchsäure, welche aus nachwachsenden Rohstoffen erhalten werden kann, erzeugt werden kann.

Ein weiterer Vorteil des erfindungsgemäßen Verfahrens besteht darin, dass je nach vorhandener Rohstoffquelle zwischen 3-Hydroxypropionsäure und Milchsäure als Rohstoff gewechselt werden kann.

Ein weiterer großer Vorteil gegenüber einer heterogenen Gasphasendehydratisierung in bzw. an der Phasengrenze der Flüssigphase ist es, dass auf eine vorherige, vollständige Verdampfung der organischen Säuren verzichtet werden kann. Bei diesen Verdampfungen kommt es, da die Verdampfungstemperatur und Zersetzungstemperatur nahe beieinander liegen, zu starker Koksbildung. Dies kann nur durch sehr viel zusätzlich zugeführtes Wasser, das einerseits verdampft und in den nachfolgenden Schritten aufwendig wieder abgetrennt werden muss, minimiert werden. Bei einer direkten Umsetzung an der Salzschmelze kann auf diese starke Verdünnung weitgehend verzichtet werden und mit konzentrierteren Lösungen gearbeitet werden

Die erfindungsgemäßen Gegenstände werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt, insbesondere in Bezug auf den Sachverhalt, in dessen Zusammenhang das Dokument zitiert wurde, vollständig zum Offenbarungsgehalt der vorliegenden Erfindung gehören. Bei Prozentangaben handelt es sich, wenn nicht anders angegeben, um Angaben in Gewichtsprozent. Werden nachfolgend Mittelwerte angegeben, so handelt es sich, wenn nicht anderes angegeben, um Gewichtsmittel. Werden nachfolgend Parameter angegeben, die durch Messung bestimmt wurden, so wurden die Messungen, wenn nicht anders angegeben, bei einer Temperatur von 25 °C und einem Druck von 101.325 Pa durchgeführt.

Das erfindungsgemäße Verfahren zur Herstellung von Acrylsäure durch Dehydratisierung einer Hydroxycarbonsäure, die 3 Kohlenstoffatome aufweist, zeichnet sich dadurch aus, dass zur Dehydratisierung die Hydroxycarbonsäure bei einer Temperatur von größer 150 °C mit einer bei dieser Temperatur flüssigen Mischung, enthaltend mindestens ein Metallsalz einer Hydroxycarbonsäure, die 3 Kohlenstoffatome aufweist, und Wasser, in Kontakt gebracht wird. Der Anteil an Wasser beträgt bezogen auf die Summe von Wasser und Metallsalz der Hydroxycarbonsäure vorzugsweise von 0,5 bis 95 Gew.-%, bevorzugt von 1 bis 80 Gew.-% und besonders bevorzugt von 2 bis 25 Gew.-%.

Die Mischung kann dadurch erzeugt werden, dass eine Lösung von Hydroxycarbonsäure, die 3 Kohlenstoffatome aufweist, und Wasser mit einem entsprechenden Metallsalz, vorzugsweise einem Metallcarbonat oder einem oder mehreren Metallsalzen der Formel (I)

M^{z+}ₓH_{3-x*z}PO₄^{z*x-} (I)

mit x = 1 oder 2, z = 1 oder 2 und M = Metall, vorzugsweise Alkali- oder Erdalkalimetall, bevorzugt Na, Ba oder K, mit der Maßgabe, dass wenn z = 2 ist x = 1 sein muss, versetzt wird. Durch die Menge des zugegebenen Metallsalzes kann eingestellt werden, ob in der Mischung noch freie Säure vorhanden ist oder nicht. Vorzugsweise wird soviel Metallsalz der Mischung zugegeben, dass das molare Verhältnis von Metallsalz der Hydroxycarbonsäure zu freier Hydroxycarbonsäure von 100 zu 1 bis 1 zu 100 beträgt. Wird als Metallsalz ein Metallcarbonat eingesetzt, beträgt das molare Verhältnis von Metallsalz der Hydroxycarbonsäure zu freier Hydroxycarbonsäure bevorzugt von 10 zu 1 bis 2 zu 1 und besonders bevorzugt von 5 zu 1 bis 3 zu 1. Wird als Metallsalz ein Metallsalz der Formel (I) eingesetzt, beträgt das molare Verhältnis von Metallsalz der Hydroxycarbonsäure zu freier Hydroxycarbonsäure bevorzugt von 1 zu 50 bis 1 zu 10 und besonders bevorzugt von 1 zu 40 bis 1 zu 20.

Vorzugsweise beträgt die Temperatur, bevorzugt die Temperatur der Mischung 160 bis 350 °C, 170 bis 300 °C und besonders bevorzugt 180 bis kleiner 200 °C, insbesondere bei Einsatz von 3-Hydroxypropionsäure als Hydroxycarbonsäure.

Das erfindungsgemäße Verfahren wird vorzugsweise unter Rühren oder Verwendung eines gleichwirkenden Mittels betrieben, um eine möglichst vollständige Durchmischung zu erreichen.

Das Metallsalz ist vorzugsweise ein Alkali- oder Erdalkalimetallsalz. Handelt es sich bei dem Metallsalz um ein Metallcarbonat, so ist das Metallsalz bevorzugt ein Alkalimetallsalz, besonderes bevorzugt ein Natrium- oder Kaliumsalz und ganz besonders bevorzugt ein Kaliumsalz. Handelt es sich bei dem Metallsalz um ein Metallsalz der Formel (I), so ist dieses bevorzugt ausgewählt aus KH₂PO₄, K₂HPO₄, Na₂HPO₄ oder BaHPO₄ oder Mischungen von einem oder mehreren vorzugsweise von zwei der genannten Salze, besonders bevorzugt Mischungen von KH₂PO₄, K₂HPO₄ oder Na₂HPO₄ mit BaHPO₄. Werden Mischungen von Metallsalzen der Formel (I) eingesetzt, so werden vorzugsweise solche Mischungen von Metallsalzen eingesetzt, bei denen die Salze in äquimolaren Verhältnisssen vorliegen. Salze der Formel (I) können z. B. wie in WO 2013/155245 A2 beschrieben hergestellt oder von den in dieser Schrift angegebenen Quellen bezogen werden.

Die eingesetzte zu dehydratisierende Hydroxycarbonsäure und die Hydroxycarbonsäure, auf welcher das Metallsalz basiert, sind vorzugsweise gleich. Sollen nacheinander unterschiedliche Hydroxycarbonsäuren als Rohstoffe eingesetzt werden, ist es vorteilhaft, die Mischung ebenfalls auszutauschen.

Die Mischung weist vorzugsweise mindestens einen Polymerisationsinhibitor (Verbindung, die die Polymerisation der Acrylsäure und/oder der eingesetzten Hydroxycarbonsäure hemmt), bevorzugt ausgewählt aus Phenotiazin oder Hydrochinon oder seine Methylether, oder Mischungen daraus, besonders bevorzugt Hydrochinon und/oder seine Methylether, ganz besonders bevorzugt Hydrochinon und p-Methoxyphenol, auf. Zusätzlich oder an Stelle der vorgenannten Polymerisationsinhibitoren kann auch ein Kupferinhibitor wie z. B. Kupfercarbamat oder auch Kupferpulver zugesetzt werden. Der Anteil der Summe der Polymerisationshemmer an der flüssigen Mischung beträgt vorzugsweise von 0,05 bis 2,5 Massen-%, bevorzugt von 0,1 bis 0,5 Massen-%.

Das erfindungsgemäße Verfahren kann bei Normaldruck oder Unterdruck durchgeführt werden. Vorzugsweise wird das Verfahren bei einem Unterdruck durchgeführt. Bevorzugt beträgt der Druck über der flüssigen Mischung kleiner 500 mbar, bevorzugt kleiner 200 mbar und besonders bevorzugt von 50 bis 100 mbar.

Das in Kontrakt bringen der zu dehydratisierenden Hydroxycarbonsäure mit der Mischung kann so erfolgen, dass die zu dehydratisierende Hydroxycarbonsäure auf oder in die Mischung geleitet wird. Vorzugsweise wird die zu dehydratisierende Hydroxycarbonsäure in die Mischung eingeleitet. Bevorzugt erfolgt die Einleitung der zu dehydratisierenden Hydroxycarbonsäure in die flüssige Mischung über eine Einleitstelle, die mindestens 5 % und höchstens 75 %, vorzugsweise 10 % bis 30 % unterhalb der Oberfläche der Mischung bezogen auf die Gesamthöhe der Flüssigkeit an dieser Stelle liegt.

Als zu dehydratisierende Hydroxycarbonsäure, die 3 Kohlenstoffatome aufweist, wird vorzugsweise 3-Hydroxypropionsäure oder Milchsäure, bevorzugt Milchsäure eingesetzt.

Das erfindungsgemäße Verfahren kann in allen dem Fachmann bekannten Apparaturen durchgeführt werden, in denen ein Fluid auf eine gewünschte Reaktionstemperatur erhitzt werden kann, wobei die Apparatur vorzugsweise mit einem Druck beaufschlagt werden kann, der ausreicht, um die Reaktionskomponenten unter den gewünschten Temperaturbedingungen flüssig zu halten.

Die zu dehydratisierende Hydroxycarbonsäure kann als Reinstoff oder in Form einer Zusammensetzung mit der Mischung in Kontakt gebracht werden. Vorzugsweise wird die zu dehydratisierende Hydroxycarbonsäure als Zusammensetzung eingesetzt. Diese Zusammensetzung weist vorzugsweise neben der zu dehydratisierenden Hydroxycarbonsäure Wasser auf. Der Gehalt an Wasser in der Zusammensetzung beträgt bezogen auf die Summe aus zu dehydratisierender Hydroxycarbonsäure und Wasser vorzugsweise von 10 bis 55, bevorzugt von 20 bis 50 Gew.-%.

Die Zusammensetzung kann des Weiteren ein Metallsalz, bevorzugt das gleiche, welches zur Herstellung der Mischung eingesetzt wurde, aufweisen. Der Anteil des Metallsalzes an der Zusammensetzung beträgt vorzugsweise von 0,01 bis 1 Gew.-%, bevorzugt von 0,2 bis 0,5 Gew.-% bezogen auf die Zusammensetzung.

Die Zusammensetzung kann außerdem Polymerisationshemmer, vorzugsweise die oben als bevorzugt angegebenen Polymerisationshemmer, aufweisen. Der Anteil der Polymerisationshemmer an der Zusammensetzung beträgt vorzugsweise von 0,05 bis 2,5 Massen-%, bevorzugt von 0,1 bis 0,5 Massen-% bezogen auf die Zusammensetzung.

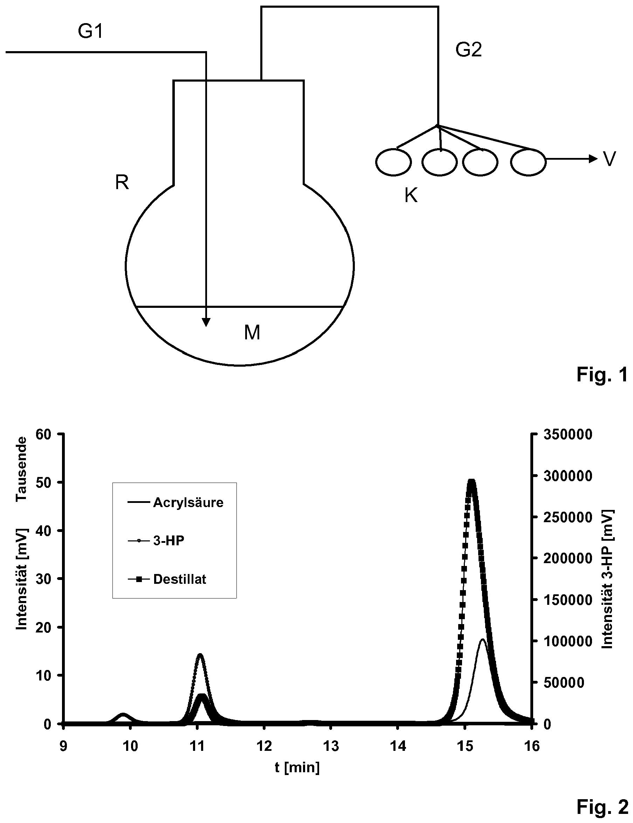

Die vorliegende Erfindung wird anhand der Figuren Fig. 1 bis Fig. 3 näher erläutert, ohne dass die Erfindung auf diese Ausführungsform beschränkt sein soll.

Fig. 1 zeigt schematisch den in Beispiel 1 verwendeten Versuchsaufbau. In einen beheizbaren Rundkolben R, der mit einem Stopfen und einem Blattrührer ausgerüstet ist, führen zwei Glasröhren. Die linke Glasröhre G1 ist so lang ausgeführt, dass sie in eine im Rundkolben befindliche flüssige Mischung hineinragt, und dient der Zuführung von Substanzen in den Rundkolben. Die rechte Glasröhre G2 ist kürzer und reicht nur in den Gasraum. Die rechte Glasröhre G2 ist mit einer Vakuumpumpe V verbunden. Zwischen Vakuumpumpe und Rundkolben ist eine Kühlfalle K vorhanden, in der fraktioniert Proben von kondensierten Substanzen, die den Rundkolben über die rechte Glasröhre verlassen, genommen werden können.

Die Figuren Fig. 1 und Fig. 2 zeigen Chromatogramme der untersuchten Proben aus den Beispielen 1 und 2.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele

### Beispiel 1: Dehydratisierung von 3-Hydroxypropionsäure (3-HP) zu Acrylsäure

In einem 500-ml-Rundkolben einer Versuchsapparatur wie in Fig. 1 dargestellt, wurden 1 mol 3-HP als 60 Gew.-%ige Lösung in Wasser, 0,4 mol K₂CO₃, 2 g Hydrochinon und 2 g p-Methoxyphenol (MEHQ) vorgelegt. Unter Rühren wurde dieses Gemisch unter Verwendung eines Heizpilzes innerhalb von einer Stunde auf 180 °C aufgeheizt und das dabei überdestillierende Wasser verworfen. Über Glasrohr G1, welches ca. 1 cm in die flüssige Mischung ragte, die an dieser Stelle eine Höhe von 5 cm aufwies, wurden mittels einer HPLC-Pumpe ca. 120 g/h einer Zusammensetzung, die 0,1 Massen-% Hydrochinon, 0,1 Massen-% MEHQ und ad 99,8 Massen-% 3-HP als 22 Gew.-%ige Lösung in Wasser enthielt, eingeleitet. Während der gesamten Versuchsdauer wurde über Glasrohr G2 mittels einer Vakuumpumpe ein Unterdruck von ca. 95 mbar angelegt. Die über G2 abgezogenen gasförmigen Stoffe wurden über einen Glaskühler (absteigender Intensivkühler) verflüssigt und fraktioniert gesammelt. Die verbleibenden dampfförmigen Leichtsieder wurden in einer Kühlfalle kondensiert. Die aufgefangenen Fraktionen wurden mittels HPLC analysiert. Verwendet wurde ein Gerät der Firma Shimadzu vom Typ Prominence mit einer Säule vom Typ Nucleogel Sugar 810 H (MACHEREY-NAGEL). Als Eluent wurde eine 5 mM H₂SO₄-Lösung verwendet (0,7 mL/min). Wie die Analyse zeigt, enthalten alle Destillate sowie die in der Kühlfalle kondensierten Dämpfe neben geringen Mengen nicht umgesetzter 3-HP sowie Wasser hauptsächlich freie Acrylsäure (Fig. 2).

### Beispiel 2: Dehydratisierung von Milchsäure zu Acrylsäure

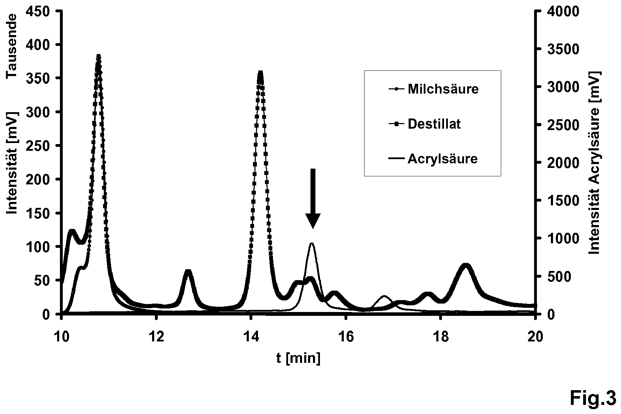

Das Beispiel 1 wurde wiederholt mit dem Unterscheid, dass eine 60 Gew.-% wässrige Lösung von Milchsäure an Stelle der wässrigen 3-HP Lösung eingesetzt wurde. Durch einen Vergleich des Chromatogramms des Destillats mit dem von Acrylsäure konnte auch für diesen Versuch die Umsetzung zu Acrylsäure nachgewiesen werden (Fig. 3).

Wie aus den HPLC-Analysen hervorgeht, konnte mit dem erfindungsgemäßen Verfahren sowohl aus 3-HP als auch aus Milchsäure Acrylsäure hergestellt werden.

### Beispiel 3: Dehydratisierung von Milchsäure zu Acrylsäure

In einem 500-ml-Rundkolben einer Versuchsapparatur wie in Fig. 1 dargestellt, der mit einer Heizmöglichkeit ausgerüstet war, wurden 1 mol Milchsäure als 90 Gew.-%ige Lösung in Wasser, 2 mol-% bezogen auf die Milchsäure an Salz, 2 g Hydrochinon und 2 g p-Methoxyphenol (MEHQ) vorgelegt. Unter Rühren wurde dieses Gemisch unter Verwendung eines Heizpilzes innerhalb von einer Stunde auf 180 °C aufgeheizt und das dabei überdestillierende Wasser verworfen. Über Glasrohr G1, welches ca. 1 cm in die flüssige Mischung ragte, die an dieser Stelle eine Höhe von 5 cm aufwies, wurden mittels einer HPLC-Pumpe ca. 120 g/h einer Zusammensetzung, die bezogen auf die Zusammensetzung 0,1 Massen-% Hydrochinon, 0,1 Massen-% MEHQ und ad 99,8 Massen-% Milchsäure als 45 Gew.-%ige Lösung in Wasser enthielt, eingeleitet. Die Sumpftemperatur wurde während der Reaktion auf ca. 300 °C eingestellt. Während der gesamten Versuchsdauer wurde über Glasrohr G2 mittels einer Vakuumpumpe ein Unterdruck von größer 100 mbar und kleiner 1 bar angelegt. Die über G2 abgezogenen gasförmigen Stoffe wurden über einen Glaskühler (absteigender Intensivkühler) verflüssigt und fraktioniert gesammelt. Die verbleibenden dampfförmigen Leichtsieder wurden in einer Kühlfalle kondensiert. Die aufgefangenen Fraktionen wurden mittels HPLC analysiert. Verwendet wurde ein Gerät der Firma Shimadzu vom Typ Prominence mit einer Säule vom Typ Nucleogel Sugar 810 H (MACHEREY-NAGEL). Als Eluent wurde eine 5 mM H₂SO₄-Lösung verwendet (0,7 mL/min). Die Ergebnisse sind in Tabelle 1 angegeben.

Die eingesetzten Salze wurden wie in WO 2013/155245 A2 hergestellt oder bei den dort angegebenen Quellen bzw. vergleichbaren Quellen in der höchsten erhältlichen Reinheit bezogen.

**Tabelle 1: Ergebnisse der Versuche in Beispiel 3**

| **Bsp.:** | **Salz** | **Sumpftemperatur** | **Reaktionszeit** | **Acrylsäure Ausbeute [mol-%]** |
|---|---|---|---|---|
| 3a | K₂HPO₄ | ∼ 300 °C | 5,3 h | 1,3 |
| 3b | KH₂PO₄ | ∼ 300 °C | 5,0 h | 0,4 |
| 3c | BaHPO₄ | ∼ 300 °C | 4,7 h | 1,2 |
| 3d | K₂HPO₄ BaHPO₄ | ∼ 300 °C | 4,4 h | 0,1 |
| 3e | KH₂PO₄ BaHPO₄ | ∼ 300 °C | 5,5 h | 0,3 |
| 3f | Na₂HPO₄ BaHPO₄ | ∼ 300 °C | 4,8 h | 0,1 |

In den Versuchen 3d bis 3f wurden Mischungen der Salze im molaren Verhältnis von 1:1 eingesetzt.

Die Versuche gemäß der Beispiel 3a bis 3f zeigen, dass eine Dehydratisierung von Milchsäure in der Flüssigphase möglich ist.

## Patentansprüche

1. Verfahren zur Herstellung von Acrylsäure durch Dehydratisierung einer Hydroxycarbonsäure, die 3 Kohlenstoffatome aufweist, **dadurch gekennzeichnet, dass** zur Dehydratisierung die Hydroxycarbonsäure bei einer Temperatur von größer 150 °C mit einer bei dieser Temperatur flüssigen Mischung, enthaltend mindestens ein Metallsalz einer Hydroxycarbonsäure, die 3 Kohlenstoffatome aufweist, und Wasser, in Kontakt gebracht wird.

2. Verfahren gemäß Anspruch 1, **dadurch gekennzeichnet, dass** die Temperatur der Mischung 160 bis 300 °C, bevorzugt 180 bis 250 °C beträgt.

3. Verfahren gemäß Anspruch 1 oder 2, **dadurch gekennzeichnet, dass** das Metallsalz ein Alkali- oder Erdalkalimetallsalz, bevorzugt ein Alkalimetallsalz und besonders bevorzugt ein Kaliumsalz ist.

4. Verfahren nach zumindest einem der Ansprüche 1 bis 3, **dadurch gekennzeichnet, dass** zur Herstellung des Metallsalzes der Hydroxycarbonsäure ein Metallcarbonat oder ein oder mehrere Metallsalze der Formel (I)
M^{z+}ₓH_{3-x*z}PO₄^{z*x-} (I)
mit x = 1 oder 2, z = 1 oder 2 und M = Metall, mit der Maßgabe, dass wenn z = 2 ist, x = 1 sein muss, eingesetzt werden.

5. Verfahren nach zumindest einem der Ansprüche 1 bis 4, **dadurch gekennzeichnet, dass** die zu dehydratisierende Hydroxycarbonsäure und die Hydroxycarbonsäure, auf welcher das Metallsalz der Hydroxycarbonsäure basiert, gleich sind.

6. Verfahren nach zumindest einem der Ansprüche 1 bis 5, **dadurch gekennzeichnet, dass** die Mischung mindestens einen Polymerisationshemmer, bevorzugt ausgewählt aus Kupferinhibitor oder Phenotiazin oder Hydrochinon oder seine Methylether, oder Mischungen daraus, besonders bevorzugt Hydrochinon und/oder seine Methylether, ganz besonders bevorzugt Hydrochinon und p-Methoxyphenol, aufweist.

7. Verfahren gemäß Anspruch 6, **dadurch gekennzeichnet, dass** der Anteil der Summe der Polymerisationshemmer an der flüssigen Mischung von 0,05 bis 2,5 Massen-%, bevorzugt von 0,1 bis 0,5 Massen-% beträgt.

8. Verfahren nach zumindest einem der Ansprüche 1 bis 7, **dadurch gekennzeichnet, dass** der Druck über der flüssigen Mischung kleiner 500 mbar, bevorzugt kleiner 200 mbar und besonders bevorzugt von 50 bis 100 mbar beträgt.

9. Verfahren nach zumindest einem der Ansprüche 1 bis 8, **dadurch gekennzeichnet, dass** das in Kontakt bringen der zu dehydratisierenden Hydroxycarbonsäure mit der Mischung so erfolgt, dass diese Hydroxycarbonsäure in die Mischung eingeleitet wird.

10. Verfahren gemäß Anspruch 9, **dadurch gekennzeichnet, dass** die Einleitung der zu dehydratisierenden Hydroxycarbonsäure in die flüssige Mischung über eine Einleitstelle erfolgt, die mindestens 5 % und höchstens 75 % unterhalb der Oberfläche der Mischung bezogen auf die Gesamthöhe der Flüssigkeit an dieser Stelle liegt.

11. Verfahren nach zumindest einem der Ansprüche 1 bis 10, **dadurch gekennzeichnet, dass** als zu dehydratisierende Hydroxycarbonsäure, die 3 Kohlenstoffatome aufweist, 3-Hydroxypropionsäure oder Milchsäure, bevorzugt Milchsäure eingesetzt wird.

## Claims

1. Method for producing acrylic acid by dehydration of a C₃ hydroxycarboxylic acid, **characterized in that** dehydration is achieved by contacting, at a temperature of more than 150°C, the hydroxycarboxylic acid with a mixture which is liquid at this temperature and comprises at least one metal salt of a C₃ hydroxycarboxylic acid, and water.

2. Method according to Claim 1, **characterized in that** the temperature of the mixture is from 160°C to 300°C, preferably from 180°C to 250°C.

3. Method according to Claim 1 or 2, **characterized in that** the metal salt is an alkali metal salt or an alkaline earth metal salt, preferably an alkali metal salt and more preferably a potassium salt.

4. Method according to at least one of Claims 1 to 3, **characterized in that** the hydroxycarboxylic acid metal salt is produced using a metal carbonate or one or more metal salts of formula (I)
M^{z+}ₓH_{3-X*z}PO₄^{z*x-} (I)
where x = 1 or 2, z = 1 or 2 and M = metal, with the proviso that when z = 2, x = 1.

5. Method according to at least one of Claims 1 to 4, **characterized in that** the hydroxycarboxylic acid to be dehydrated and the hydroxycarboxylic acid on which the hydroxycarboxylic acid metal salt is based are identical.

6. Method according to at least one of Claims 1 to 5, **characterized in that** the mixture comprises at least one polymerization inhibitor, preferably selected from copper inhibitor, phenothiazine, hydroquinone and its methyl ethers, and mixtures thereof, more preferably hydroquinone and/or its methyl ethers, most preferably hydroquinone and p-methoxyphenol.

7. Method according to Claim 6, **characterized in that** the fraction of the sum total of polymerization inhibitors in the liquid mixture is from 0.05% to 2.5% by mass, preferably from 0.1% to 0.5% by mass.

8. Method according to at least one of Claims 1 to 7, **characterized in that** the pressure above the liquid mixture is below 500 mbar, preferably below 200 mbar and more preferably from 50 to 100 mbar.

9. Method according to at least one of Claims 1 to 8, **characterized in that** the contacting of the hydroxycarboxylic acid to be dehydrated and the mixture is performed such that the hydroxycarboxylic acid is introduced into the mixture.

10. Method according to Claim 9, **characterized in that** the introduction of the hydroxycarboxylic acid to be dehydrated into the liquid mixture is performed via an introduction point disposed no less than 5% and no more than 75% below the surface of the mixture based on the total height of the liquid at this point.

11. Method according to at least one of Claims 1 to 10, **characterized in that** the employed C₃ hydroxycarboxylic acid to be dehydrated is 3-hydroxypropionic acid or lactic acid, preferably lactic acid.

## Revendications

1. Procédé de fabrication d'acide acrylique par déshydratation d'un acide hydroxycarboxylique, qui comprend 3 atomes de carbone, **caractérisé en ce que**, pour la déshydratation, l'acide hydroxycarboxylique est mis en contact à une température de plus de 150 °C avec un mélange liquide à cette température, contenant au moins un sel métallique d'un acide hydroxycarboxylique, qui comprend 3 atomes de carbone, et de l'eau.

2. Procédé selon la revendication 1, **caractérisé en ce que** la température du mélange est de 160 à 300 °C, de préférence de 180 à 250 °C.

3. Procédé selon la revendication 1 ou 2, **caractérisé en ce que** le sel métallique est un sel de métal alcalin ou alcalino-terreux, de préférence un sel de métal alcalin et de manière particulièrement préférée un sel de potassium.

4. Procédé selon au moins l'une quelconque des revendications 1 à 3, **caractérisé en ce que**, pour la fabrication du sel métallique de l'acide hydroxycarboxylique, un carbonate métallique ou un ou plusieurs sels métalliques de formule (I)
M^{z+}ₓH_{3-x*z}PO₄^{z*x-} (I)
avec x = 1 ou 2, z = 1 ou 2, et M = un métal, à condition que lorsque z = 2, x doit être = 1, sont utilisés.

5. Procédé selon au moins l'une quelconque des revendications 1 à 4, **caractérisé en ce que** l'acide hydroxycarboxylique à déshydrater et l'acide hydroxycarboxylique à la base du sel métallique d'acide hydroxycarboxylique sont identiques.

6. Procédé selon au moins l'une quelconque des revendications 1 à 5, **caractérisé en ce que** le mélange comprend au moins un inhibiteur de polymérisation, de préférence choisi parmi un inhibiteur à base de cuivre ou la phénothiazine ou l'hydroquinone ou son éther méthylique, ou leurs mélanges, de manière particulièrement préférée l'hydroquinone et/ou son éther méthylique, de manière tout particulièrement préférée l'hydroquinone et le p-méthoxyphénol.

7. Procédé selon la revendication 6, **caractérisé en ce que** la proportion de la somme des inhibiteurs de polymérisation dans le mélange liquide est de 0,05 à 2,5 % en masse, de préférence de 0,1 à 0,5 % en masse.

8. Procédé selon au moins l'une quelconque des revendications 1 à 7, **caractérisé en ce que** la pression sur le mélange liquide est inférieure à 500 mbar, de préférence inférieure à 200 mbar et de manière particulièrement préférée de 50 à 100 mbar.

9. Procédé selon au moins l'une quelconque des revendications 1 à 8, **caractérisé en ce que** la mise en contact de l'acide hydroxycarboxylique à déshydrater avec le mélange a lieu par introduction de cet acide hydroxycarboxylique dans le mélange.

10. Procédé selon la revendication 9, **caractérisé en ce que** l'introduction de l'acide hydroxycarboxylique à déshydrater dans le mélange liquide a lieu par un emplacement d'introduction, qui se situe au moins 5 % et au plus 75 % en dessous de la surface du mélange par rapport à la hauteur totale du liquide à cet emplacement.

11. Procédé selon au moins l'une quelconque des revendications 1 à 10, **caractérisé en ce que** l'acide 3-hydroxypropionique ou l'acide lactique, de préférence l'acide lactique, est utilisé en tant qu'acide hydroxycarboxylique à déshydrater qui comprend 3 atomes de carbone.
